# EUROPEAN PATENT APPLICATION

(11) **EP 4 752 546 A1**
(43) Date of publication of application: **03.06.2026**
(21) Application number: 25218276.1
(22) Date of filing: 25.11.2025
(51) Int. Cl.: G01N 33/543, G01N 33/551

(54) **PARTICLE FOR IMMUNOAGGLUTINATION ASSAY AND TEST REAGENT FOR IN VITRO DIAGNOSIS**

(30) Priority: 29.11.2024 JP 2024208060
(71) Applicant: Canon Kabushiki Kaisha, Tokyo, 146-8501 (JP)
(72) Inventor: KANAZAKI, Kengo, Tokyo (JP); SAKAKIBARA, Teigo, Tokyo (JP); KASAI, Gota, Tokyo (JP)
(74) Representative: WESER & Kollegen Patentanwälte PartmbB

(57) **Abstract**

There is provided a particle for the immunoagglutination assay that is a particle for immunoagglutination assay, which includes a diamond particle and a ligand that specifically binds to a biological substance. The ligand is immobilized to the diamond particle, and the particle for the immunoagglutination assay has a particle size of 50 nm or more and 1,000 nm or less.

## Description

### TECHNICAL FIELD

The present disclosure relates to a particle for an immunoagglutination assay and a test reagent for in vitro diagnosis.

### BACKGROUND

As a method of detecting a target substance in a biological sample (hereinafter, referred to as specimen) in the clinical testing field, a method using an immunological agglutination (hereinafter, may be referred to as an immunoagglutination assay) is known. In this method, a dispersion of particles bound (sensitized) with a ligand such as an antibody or an antigen is mixed with a specimen which may contain a biological substance (such as an antigen and an antibody) as a detection target. If a specimen contains the biological substance as a detection target, the particles sensitized with a ligand cause agglutination. Accordingly, it is possible to identify the presence or absence of, or quantify the detection target substance, by measuring this agglutination as the amount of the change in the light intensity such as scattered light intensity, transmitted light intensity, or absorbance.

Japanese Patent Laid-Open No. 4-218772 provides a particle including a copolymer of styrene and glycidyl (meth)acrylate as a particle that is used in immunological agglutination. Known particles including organic polymers have room for improvement in the detection sensitivity of biological substances as detection targets.

### SUMMARY

The present disclosure is directed to provide a particle for an immunoagglutination assay with improved detection sensitivity of a biomolecule as a detection target in a method using immunological agglutination.

The present disclosure in its first aspect provides a particle for an immunoagglutination assay as specified in claim 1. Optional features are specified in claims 2 to 9.

The present disclosure in its second aspect provides a test reagent for in vitro diagnosis as specified in claim 10. Optional features are specified in claims 11 and 12.

The present disclosure in its third aspect provides an examination kit for in vitro diagnosis as specified in claim 13.

Features of the present disclosure will become apparent from the following description of embodiments with reference to the attached drawings. The following description of embodiments is described by way of example.

### BRIEF DESCRIPTION OF THE DRAWING

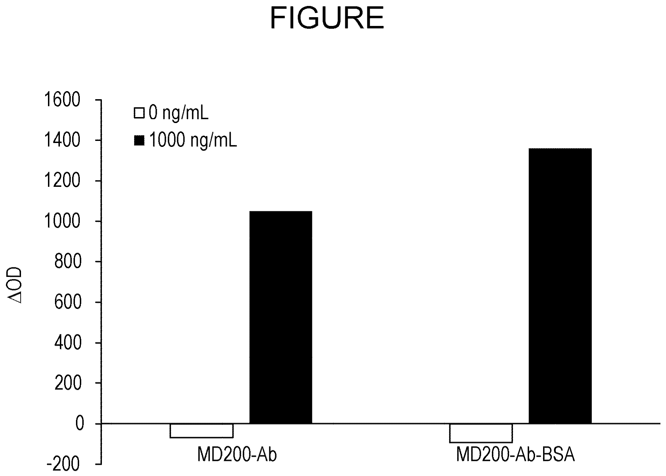

Fig. 1 is a graph showing the results of detection of ferritin using a reagent including a particle for the immunoagglutination assay according to an Example of the present disclosure.

### DESCRIPTION OF THE EMBODIMENTS

Embodiments of the present disclosure will be described in detail below, but the technical scope of the present disclosure is not limited the embodiments.

### Particle for immunoagglutination assay

The particle for the immunoagglutination assay according to the present embodiment includes a diamond particle and a ligand that specifically binds to a biological substance. The ligand is immobilized to the diamond particle, and the particle for the immunoagglutination assay has a particle size of 50 nm or more and 1,000 nm or less. In the immunoagglutination assay, an increase in the scattered light or a decrease in the transmitted light due to particle agglutination caused by immunoagglutination is detected. The transmitted light decreases with an increase in the concentration of the particle or the optical path length or improvement in the scattering efficiency. That is, an example of the method for improving the detection sensitivity in the immunoagglutination assay can be an increase in the scattering efficiency of the particle. An example of the method for improving the scattering efficiency can be an increase in the refractive index of the particle. The particle for the immunoagglutination assay in the present embodiment includes a diamond particle having a nanometer-order particle size (nanometer diamond particle), and the nanometer diamond particle has a refractive index of 2.4 or more. This refractive index is very high compared to those of particles including organic polymers that are generally used in the immunoagglutination assay. Consequently, the scattering efficiency of light incident on the particles for the immunoagglutination assay is high, and the amount of the change in the light intensity is large when a biological substance as a detection target is detected. Accordingly, the particle for the immunoagglutination assay according to the present embodiment can detect a biological substance as a detection target with high sensitivity in the immunoagglutination assay. The immunoagglutination assay can also be referred to as immunonephelometry or a latex immunoagglutination assay.

When the diamond particle in the present embodiment is non-fluorescent, the light incident on the diamond particle is advantageously used for decreasing the transmitted light due to agglutination of the particles without being used in fluorescence emission. Here, the non-fluorescent diamond particle means that when the diamond particle is excited, fluorescence with a wavelength of 400 nm or more and 1,000 nm or less is not emitted. The excitation light that is used here is light with a wavelength of 400 nm or more and 1,000 nm or less. Not emitting fluorescence with a wavelength of 400 nm or more and 1,000 nm or less includes emitting a very small amount of fluorescence that does not affect the improvement in the sensitivity in the immunoagglutination assay. That is not substantially emitting fluorescence. Accordingly, when the diamond particle is non-fluorescent, in agglutinated and unagglutinated particles for the immunoagglutination assay, the difference in the amount of decrease in the transmitting light (transmitted light) of light incident on the particles for the immunoagglutination assay is large. This is because if the energy of incident light is used for fluorescence emission, regardless of the presence or absence of agglutination, the energy of transmitted light is decreased. Accordingly, the diamond particle in the present embodiment can be non-fluorescent. In particular, when the wavelength of the light that is used in the immunoagglutination assay is the same as that of excitation light, the diamond particle can be non-fluorescent.

### Immobilization of ligand to diamond particle

In the present embodiment, the ligand is immobilized to the diamond particle by at least anyone of a covalent bond, an ionic bond, a hydrogen bond, and hydrophobic interaction. The ionic bond, the hydrogen bond, and the hydrophobic interaction can be categorized as non-covalent bonds. The diamond particle in the present embodiment may be modified by a compound having at least any one functional group of a carboxy group, a hydroxy group, and an amino group. In such a case, the ligand can be immobilized to the diamond particle through a reaction of the ligand with these functional groups. The compound having at least one functional group of a hydroxy group, a carboxy group, and an amino group can modify mainly the surface of the diamond particle by a known chemical reaction.

### Particle size

The particle size of the particle for the immunoagglutination assay in the present embodiment is the number average particle size in water and is 50 nm or more or 1,000 nm or less. The particle size of the particle for the immunoagglutination assay in the present embodiment can be 100 nm or more and 500 nm or less.

When the particle size of the particle for the immunoagglutination assay is 100 nm or more and 500 nm or less, the handling in a centrifugation procedure is excellent, and the specific surface area is large. In the present embodiment, the number average particle size can be measured by a dynamic light scattering method. Specifically, a dispersion of a target particle is measured by a dynamic light scattering, the obtained light intensity is converted into number distribution, and the average value thereof can be defined as the number average particle size.

### Ligand

The ligand in the present embodiment is a binding material that specifically binds to the biological substance as a detection target.

The ligand in the present embodiment can be anyone of an antibody, an antigen, a fragmented antibody, and a VHH (variable domain of heavy chain of heavy chain antibody) antibody.

When the diamond particle according to the present embodiment is modified with a compound having a carboxy group, a ligand can be immobilized to the carboxy group. The particle obtained by immobilizing a ligand to the diamond particle according to the present embodiment can be called a ligand-sensitized particle. The immobilization of a ligand to the diamond particle in the present embodiment can be called ligand sensitization.

The ligand in the present embodiment is a compound that specifically binds to a receptor having a specific target substance. The site of a ligand that binds to a target substance is defined and has high selective or specific affinity. Examples of the ligand include an antigen and an antibody, an enzyme protein and its substrate, a hormone or a signal substance such as a neurotransmitter and its receptor, and a nucleic acid, but the ligand of the present embodiment is not limited thereto. The particle for the immunoagglutination assay in the present embodiment can also be expressed as a ligand-sensitized particle having high selective or specific affinity to a target substance.

The method for chemically reacting a ligand with the carboxy group possessed by the particle for the immunoagglutination assay according to the present embodiment can be a known method as long as the purpose of the present disclosure can be achieved. For example, a carbodiimide-mediated reaction and an NHS ester activation reaction are chemical reactions that are often used. However, the method for chemical reaction of a carboxy group and a ligand in the present embodiment is not limited thereto.

In the ligand-sensitized particle of the present embodiment, the carboxy group remained without binding with the ligand may be subjected to active esterification and be bound to a hydrophilic molecule. This is generally called, for example, active ester inactivation or carboxy group blocking or masking treatment and is performed for decreasing non-specific absorption of protein to the carboxy group or improving dispersion stability of the ligand-sensitized particle. In the present embodiment, the hydrophilic molecule can be polyethylene glycol (PEG) or trishydroxymethylaminomethane (Tris). PEG can be used because absorption of protein to a particle for immunoagglutination assay can be highly decreased by binding PEG to the particle. As is described later in Examples, when active ester inactivation is performed using PEG, the molecular weight of PEG is important, and a large molecular weight may inhibit the antigen-antibody reaction. Accordingly, the molecular weight of PEG is 350 or more and 5,000 or less and 1,000 or more and 2,000 or less. The PEG in the present embodiment can be PEG having a functional group that has reactivity to a carboxy group or active ester, for example, PEG having an amino group and PEG having a primary amine. PEG may be a liner polymer or may be a branched polymer. The Tris is represented by the following formula (1), and examples of PEG are represented by the following formulae (2) and (3). In the following formulae (2) and (3), n represents the number of oxyethylene units and is an integer of 1 or more and 50 or less:

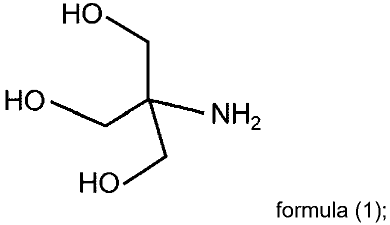

CH₃O-(CH₂CH₂O)ₙ-CH₂CH₂NH₂ formula (2);

and

CH₃O-(CH₂CH₂O)ₙ-CH₂CH₂CH₂NH₂ formula (3).

That is, in the particle for the immunoagglutination assay according to the present embodiment, the functional group is a carboxy group, and the constitution of the particle includes a structure in which an esterified carboxy group and a compound represented by anyone of the structural formulae (1), (2), and (3) are bound to each other.

The amount of a ligand to be bound is also an important factor, and a small bound amount (also can be called immobilization amount) of a ligand decreases the activity between an antigen and an antibody and is therefore not preferred. A large bound amount of a ligand also causes a deterioration in the dispersibility of the ligand-sensitized particle. Although it depends on the particle size, if the average particle size is about 200 nm, 1 mg of the diamond particle can be reacted to 1 µg or more and 500 µg or less of a ligand or 10 µg or more and 200 µg or less of a ligand.

The particle for immunoagglutination assay in the present embodiment can be applied to the immunoagglutination assay (latex immunoagglutination assay) that uses an antibody or antigen as the ligand and is widely used in fields such as a clinical test and biochemistry research. When a general particle is applied to the latex immunoagglutination assay, an antigen (antibody) as a target substance and foreign substances in serum are non-specifically absorbed to the particle surface. Consequently, unintended particle agglutination is detected, which interferes with accurate measurement problematically. Accordingly, for the purpose of decreasing false-positive noises, in general, a particle is coated with a biological substance such as albumin as a blocking reagent to reduce non-specific absorption to the particle surface and is then used. For example, bovine serum albumin (BSA) may be present (coated) on the surface of a particle for the immunoagglutination assay in the present embodiment.

### Test reagent

The test reagent according to the present embodiment includes a particle (ligand-sensitized particle) for the immunoagglutination assay according to the present embodiment and a dispersion medium for dispersing the particle for the immunoagglutination assay. The amount of the ligand-sensitized particle contained in the test reagent in the present embodiment can be 0.001 mass% or more and 20 mass% or less and can be from 0.01 mass% to 10 mass%. The test reagent according to the present embodiment may further include a third substance such as a solvent or a blocking reagent, in addition to the ligand-sensitized particle according to the present embodiment, within a range that can achieve the purpose of the present disclosure. The third substance such as a solvent and a blocking reagent may include two or more types in combination. The solvent that is used in the present disclosure is, for example, at least anyone of a phosphate buffer solution, a glycine buffer solution, a Good's buffer solution, a Tris buffer solution, and an ammonia buffer solution, but the solvent contained in the test reagent according to the present embodiment is not limited thereto.

### Examination kit

The examination kit for in vitro diagnosis in the present embodiment includes a test reagent according to the present embodiment and a casing containing the test reagent. The examination kit according to the present embodiment can further include a reaction buffer solution containing albumin (hereinafter, reagent 2), in addition to the test reagent (hereinafter, reagent 1) according to the present embodiment. Examples of the albumin include serum albumin, and the albumin may be protease-treated albumin. The amount of the albumin contained in the reagent 2 is from 0.001 mass% to 5 mass% as a guideline, but the examination kit in the present embodiment is not limited thereto. Both of the reagent 1 and the reagent 2 or anyone of them may contain a sensitizer for the latex immunoagglutination assay. Examples of the sensitizer for the latex immunoagglutination assay include, but are not limited to, polyethylene glycol, polyvinyl alcohol, polyvinylpyrrolidone, and polyalginic acid. Both of the reagent 1 and the reagent 2 or anyone of them may contain a surfactant. Since the surfactant has an effect of stabilizing the particle and protein, for example, polyoxyethylene sorbitan monolaurate and poly(oxyethylene)octylphenyl ether are suitably used. The examination kit in the present embodiment may include a positive control, a negative control, a serum diluent, and so on, in addition to the reagent 1 and the reagent 2. As the medium of the positive control and the negative control, serum not containing a measurable target substance, physiological saline, or a solvent may be used.

### Detection method

The method of detecting a target substance in a specimen by the latex immunoagglutination assay of the present embodiment is characterized by mixing the ligand-sensitized particle for latex immunoagglutination of the present embodiment and a specimen that may contain a target substance. Mixing of the ligand-sensitized particle for latex immunoagglutination of the present embodiment and a specimen can be performed in a range of from pH 3.0 to pH 11.0. The mixing temperature is in a range of from 20°C to 50°C, and the mixing time is in a range of from 10 seconds to 30 minutes. The concentration of the ligand-sensitized particle for latex immunoagglutination of the present embodiment in the detection method of the present embodiment can be from 0.001 mass% to 5 mass% and can be from 0.01 mass% to 1 mass% in the reaction system. The detection method of the present embodiment optically detects the agglutination generated as a result of mixing of the ligand-sensitized particle for latex immunoagglutination of the present embodiment and a specimen. Specifically, a target substance in a specimen can be detected by optically detecting agglutination, and the concentration of the target substance can be further measured. As the method for optically detecting agglutination, the amount of the change in scattered light intensity, transmitted light intensity, absorbance, or the like may be measured using an optical apparatus that can detect them.

### Examples

The present disclosure will be described in detail with reference to Examples below, but the present disclosure is not limited to these Examples.

### Example 1

Diamond powder MD200 (manufactured by Tomei Diamond Corporation) was dissolved in 10 mM 2-[4-(2-hydroxyethyl)-1-piperazinyl]-ethanesulfonic acid (HEPES) buffer solution (pH 7.9) to obtain a 10 mg/mL diamond particle dispersion. Subsequently, an anti-ferritin antibody (manufactured by Mikuri Immunolab.) was dissolved in 10 mM HEPES (pH 7.9) to a concentration of 5.6 mg/mL. The above dispersion and solutions (200 µL each) were mixed, and the mixture was shaken at 4°C overnight. The resulting mixture was divided into two portions of 200 µL each, and 120 µL of a 10 mg/mL BSA solution (manufactured by Sigma-Aldrich Japan, dissolved in 10 mM HEPES, pH 7.9) was added to one of the portions, followed by shaking at 4°C overnight. Subsequently, the solution without BSA and the solution with BSA were subjected to centrifugation. The ligand-sensitized particles (particles for the immunoagglutination assay) were collected, and the supernatants were discarded. The collected ligand-sensitized particles were redispersed in 10 mM HEPES (pH 7.9), and the resulting dispersions were subjected to centrifugation again. The collection of ligand-sensitized particles with a centrifugation and the redispersion in 10 mM HEPES (pH 7.9) were repeated twice.

Regarding the obtained particle suspensions, the particle without BSA is referred to as MD200-Ab, and the particle with BSA is referred to as MD200-Ab-BSA. The average particle sizes of the obtained particles for the immunoagglutination assay evaluated by DLS (dynamic light scattering) were 234 nm and 387 nm, respectively.

Subsequently, the obtained MD200-Ab was diluted to 0.01%, and the absorbance thereof was measured. In the measurement of the absorbance, an ultraviolet and visible spectrophotometer (BioSpectrometer kinetic, Eppendorf) was used, the sample was injected into a plastic cell with an optical path length of 10 mm. As a comparison target, a synthetic polystyrene particle (particle size: about 300 nm) was similarly diluted to 0.01%, and the absorbance was measured. As a result, the absorbance of MD200-Ab was 1.5, and the absorbance of the synthetic polystyrene particle was 0.5. This demonstrates that the particle for the immunoagglutination assay according to this Example had high scattering efficiency compared to the synthetic polystyrene particle. That is, it can be expected to detect a biological substance as a detection target with high sensitivity by the immunoagglutination assay.

Subsequently, sensitization (immobilization) of the anti-ferritin antibody to the diamond particle was confirmed by protein quantification. Specifically, the method reacted the ligand-sensitized particle (particle for the immunoagglutination assay) and a BCA reagent. First, 25 µL (amount of the particle: 25 µg) of the dispersion (0.1% solution) of the ligand-sensitized particle was fractionated. Separately, 7 mL of A solution and 140 µL of B solution of a protein assay BCA kit (FUJIFILM Wako Pure Chemical Corporation) were mixed to prepare an AB solution. The AB solution (200 µL) was added to the particle solution (25 µL), followed by incubation at 60°C for 30 minutes.

The resulting solution was centrifugated at 15,000 rpm (20,400 g) for 5 minutes at 4°C, and 200 µL of the supernatant was collected with a pipette. Absorbances thereof and of standard samples (antibody diluted with 10 mM HEPES to several concentrations within a range of 0 to 200 µg/mL) were measured at 562 nm with a multi-mode microplate reader (Synergy MX, BioTek). The amount of the antibody was calculated from the resulting standard curve.

The amount of an antibody sensitized to particles (antibody binding amount per particle weight (immobilized amount of antibody) (µg/mg)) was determined by dividing the calculated antibody amount by the particle weight (here, 0.025 mg). As a result, the sensitizing amount of the antibody of MD200-Ab was 84 µg/mg, and it was confirmed that the particles were sensitized with the anti-ferritin antibody.

The sensitivity of the ligand-sensitized particle (particle for the immunoagglutination assay) was evaluated by the latex immunoagglutination assay (immunoagglutination assay). Specifically, the method included reacting antibody-sensitized particles to an antigen to form aggregate of an immunocomplex, irradiating the aggregate with light, and measuring the attenuation (absorbance) of irradiation light by scattering with an absorption spectrometer. The proportion of the aggregate increases depending on the amount of the antigen contained in a specimen to increase the absorbance. In the evaluation of sensitivity, it is desirable that the increase in the absorbance (described by ΔOD × 10,000) at a predetermined antigen concentration is large. The absorbance was measured using the above-mentioned ultraviolet and visible spectrophotometer and setting a sample in a plastic cell with an optical path length of 10 mm. The measuring method will be described specifically below.

Specifically, a ferritin solution (ferritin concentration: 0 ng/mL or 1,000 ng/mL, 15 µL) and 10 mM HEPES (pH 7.9, 15 µL) were mixed in a plastic cell and were heated at 37°C for 5 minutes (hereinafter, referred to as a sample). Dispersion solutions of ligand-sensitized particles (MD200-Ab and MD200-Ab-BSA) (particle concentration: 0.01 wt%, 10 mM HEPES, pH 7.9, 30 µL each) were added to the above sample, and pipetting was quickly performed carefully so as not to cause air bubbles in order to obtain sample-particle liquid mixtures. The absorbance of the sample-particle liquid mixture at 572 nm was read as Abs 1. The sample-particle liquid mixture was heated at 37°C for 5 minutes, and the absorbance at 572 nm was then read as Abs 2. The value obtained by subtracting Abs 1 from Abs 2 was multiplied by 10,000 to obtain the ΔOD × 10,000 value.

The results are shown in Fig. 1. In the ligand-sensitized particle (particle for the immunoagglutination assay) of the present Example, an increase in the ΔOD × 10,000 value was observed in the presence of ferritin. This resulted from binding of ferritin as an antigen to the ligand-sensitized particle to form particle aggregate, and it was demonstrated that the ligand-sensitized particle functions as a particle to be used in the latex immunoagglutination assay (immunoagglutination assay).

Disclosure of the present embodiment includes the following constitution and method:
(Configuration 1) A particle for an immunoagglutination assay, comprising:
   a diamond particle; and
   a ligand that specifically binds to a biological substance,
   wherein
   the ligand is immobilized to the diamond particle, and
   the particle for the immunoagglutination assay has a particle size of 50 nm or more and 1,000 nm or less;
(Configuration 2) The particle for the immunoagglutination assay according to configuration 1, wherein the ligand is immobilized to the diamond particle by at least anyone of a covalent bond, an ionic bond, a hydrogen bond, and hydrophobic interaction;
(Configuration 3) The particle for the immunoagglutination assay according to configuration 1 or 2, wherein when the diamond particle is excited by light having a wavelength of 400 or more and 1,000 nm or less, the diamond particle does not emit fluorescence having a wavelength of 400 nm or more and 1,000 nm or less;
(Configuration 4) The particle for the immunoagglutination assay according to any one of configurations 1 to 3, wherein the diamond particle is modified by a compound comprising at least any one functional group selected from a carboxy group, a hydroxy group, and an amino group, and the ligand is immobilized to the diamond particle by a reaction between the ligand and the functional group;
(Configuration 5) The particle for the immunoagglutination assay according to configuration 4, wherein the functional group is a carboxy group, and the carboxy group is esterified and is bound to a compound represented by any of the following formulae (1), (2), and (3):

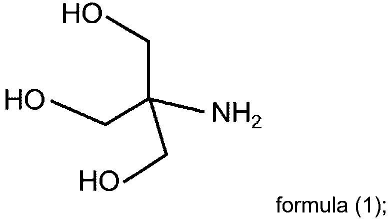

   CH₃O-(CH₂CH₂O)ₙ-CH₂CH₂NH₂ formula (2);

   and

   CH₃O-(CH₂CH₂O)ₙ-CH₂CH₂CH₂NH₂ formula (3),

   in the formula (2) and the formula (3), n is an integer of 1 or more and 50 or less;
(Configuration 6) The particle for the immunoagglutination assay according to any one of configurations 1 to 5, wherein the particle for the immunoagglutination assay has a particle size of 100 nm or more and 500 nm or less;
(Configuration 7) The particle for the immunoagglutination assay according to any one of configurations 1 to 6, wherein the ligand comprises anyone of an antibody, an antigen, a fragmented antibody, and a VHH (variable domain of heavy chain of heavy chain antibody) antibody;
(Configuration 8) The particle for the immunoagglutination assay according to any one of configurations 1 to 7, wherein the ligand comprises an anti-ferritin antibody;
(Configuration 9) The particle for the immunoagglutination assay according to any one of configurations 1 to 8, wherein bovine serum albumin is present on a surface of the particle for the immunoagglutination assay;
(Configuration 10) A test reagent for in vitro diagnosis, comprising the particle for the immunoagglutination assay according to any one of configurations 1 to 9 and a dispersion medium for dispersing the particle for the immunoagglutination assay;
(Configuration 11) The test reagent for in vitro diagnosis according to configuration 10, wherein an amount of the particle for the immunoagglutination assay included in the test reagent is 0.001 mass% or more and 20 mass% or less;
(Configuration 12) The test reagent for in vitro diagnosis according to configuration 10 or 11, further comprising at least anyone of a phosphate buffer solution, a glycine buffer solution, a Good's buffer solution, a Tris buffer solution, and an ammonia buffer solution; and
(Configuration 13) An examination kit for in vitro diagnosis, comprising the test reagent according to any one of configurations 10 to 12 and a casing containing the test reagent.

Based on the particle for the immunoagglutination assay according to the present disclosure, the amount of the change in the light intensity caused by immunological agglutination can be increased, and the detection sensitivity of a biological substance as a detection target can be improved.

While the present disclosure has been described with reference to embodiments, it is to be understood that the present disclosure is not limited to the disclosed embodiments. The scope of the following claims is to be accorded the broadest interpretation so as to encompass all such modifications and equivalent structures and functions.

Various embodiments have been described in detail above but it will be understood that the present disclosure is not limited to these embodiments and encompasses all modifications, variants, alternatives and equivalents falling within the scope of the appended claims.

## Claims

1. A particle for an immunoagglutination assay, comprising:
a diamond particle; and
a ligand that specifically binds to a biological substance,
wherein
the ligand is immobilized to the diamond particle, and
the particle for the immunoagglutination assay has a particle size of 50 nm or more and 1,000 nm or less.

2. The particle for the immunoagglutination assay according to claim 1, wherein the ligand is immobilized to the diamond particle by at least anyone of a covalent bond, an ionic bond, a hydrogen bond, and hydrophobic interaction.

3. The particle for the immunoagglutination assay according to claim 1 or 2, wherein when the diamond particle is excited by light having a wavelength of 400 or more and 1,000 nm or less, the diamond particle does not emit fluorescence having a wavelength of 400 nm or more and 1,000 nm or less.

4. The particle for the immunoagglutination assay according to any one of claims 1 to 3, wherein
the diamond particle is modified by a compound comprising at least any one functional group selected from a carboxy group, a hydroxy group, and an amino group, and
the ligand is immobilized to the diamond particle by a reaction between the ligand and the functional group.

5. The particle for the immunoagglutination assay according to claim 4, wherein
the functional group is a carboxy group, and
the carboxy group is esterified and is bound to a compound represented by any of the following formulae (1), (2), and (3): 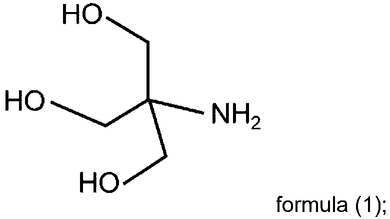
CH₃O-(CH₂CH₂O)ₙ-CH₂CH₂NH₂ formula (2);
and
CH₃O-(CH₂CH₂O)ₙ-CH₂CH₂CH₂NH₂ formula (3),
in the formula (2) and the formula (3), n is an integer of 1 or more and 50 or less.

6. The particle for the immunoagglutination assay according to any one of claims 1 to 5, wherein
the particle for the immunoagglutination assay has a particle size of 100 nm or more and 500 nm or less.

7. The particle for the immunoagglutination assay according to any one of claims 1 to 6, wherein
the ligand comprises anyone of an antibody, an antigen, a fragmented antibody, and a VHH, (variable domain of heavy chain of heavy chain antibody) antibody.

8. The particle for the immunoagglutination assay according to any one of claims 1 to 7, wherein
the ligand comprises an anti-ferritin antibody.

9. The particle for the immunoagglutination assay according to any one of claims 1 to 8, wherein
bovine serum albumin is present on a surface of the particle for the immunoagglutination assay.

10. A test reagent for in vitro diagnosis, comprising:
the particle for the immunoagglutination assay according to any one of claims 1 to 9; and
a dispersion medium for dispersing the particle for the immunoagglutination assay.

11. The test reagent for in vitro diagnosis according to claim 10, wherein
an amount of the particle for the immunoagglutination assay included in the test reagent is 0.001 mass% or more and 20 mass% or less.

12. The test reagent for in vitro diagnosis according to claim 10 or 11, further comprising:
at least anyone of a phosphate buffer solution, a glycine buffer solution, a Good's buffer solution, a Tris buffer solution, and an ammonia buffer solution.

13. An examination kit for in vitro diagnosis, comprising:
the test reagent according to any one of claims 10 to 12; and
a casing containing the test reagent.
